# EUROPÄISCHE PATENTSCHRIFT

(11) **EP 0 570 780 B1**
(45) Veröffentlichungstag und Bekanntmachung des Hinweises auf die Patenterteilung: **06.03.1996**
(21) Anmeldenummer: 93107419.9
(22) Anmeldetag: 07.05.1993
(51) Int. Cl.: C07C 317/24

(54) **Chlorethylsulfonylbenzaldehyde**
Chloroethylsulphonylbenzaldehydes
Chloroéthylsulfonylbenzaldéhydes

(30) Priorität: 20.05.1992 DE 4216590
(43) Veröffentlichungstag der Anmeldung: 24.11.1993
(73) Patentinhaber: BASF Aktiengesellschaft, D-67063 Ludwigshafen (DE)
(72) Erfinder: Marschner, Claus, Dr., W-6720 Speyer (DE); Patsch, Manfred, Dr., W-6706 Wachenheim (DE)

(56) Entgegenhaltungen:
- EP-A- 0 309 839
- EP-A- 0 587 030

## Beschreibung

Die vorliegende Erfindung betrifft neue Chlorethylsulfonylbenzaldehyde der Formel I 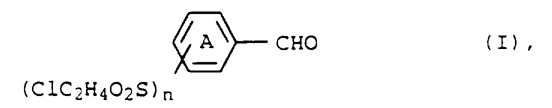 in der
n 1 oder 2 bedeutet und der Ring A substituiert sein kann, sowie ein Verfahren zu ihrer Herstellung.

Aus der US-A-4 922 014 ist die Herstellung von 2-Hydroxyethylmercaptobenzaldehyden bekannt. Diese werden durch Umsetzung von Halogenbenzaldehyden mit 2-Mercaptoethanol erhalten. Aus ihnen können durch Oxidation mit Wasserstoffperoxid in Gegenwart von Wolframverbindungen 2-Hydroxyethylsulfonylbenzaldehyde hergestellt werden.

Aufgabe der vorliegenden Erfindung war es, neue vorteilhafte Zwischenprodukte für die Herstellung von Reaktivfarbstoffen bereitzustellen. Die neuen Zwischenprodukte sollten auf einfache Weise und in guter Ausbeute herstellbar sein.

Demgemäß wurden die eingangs naher bezeichneten Chlorethylsulfonylbenzaldehyde der Formel I gefunden.

Wenn der Ring A in Formel I Substituenten trägt, so kannen dafür z.B. Halogen, wie Fluor, Chlor, Brom oder Iod, C₁-C₄-Alkyl, wie Methyl, Ethyl, Propyl, Isopropyl, Butyl, Isobutyl oder sec-Butyl, C₁-C₄-Alkoxy, wie Methoxy, Ethoxy, Propoxy, Isopropoxy, Butoxy, Isobutoxy oder sec-Butoxy, Nitro, Hydroxysulfonyl oder C₁-C₄-Alkylsulfonyl, wie Methylsulfonyl, Ethylsulfonyl, Propylsulfonyl, Isopropylsulfonyl oder Butylsulfonyl, in Betracht kommen. Der Ring A ist dabei in der Regel ein- bis dreifach, vorzugsweise ein- bis zweifach und insbesondere einfach substituiert.

Bevorzugt sind Chlorethylsulfonylbenzaldehyde der Formel Ia oder Ib 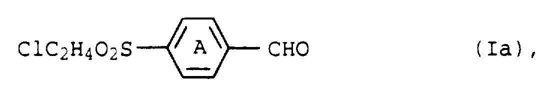 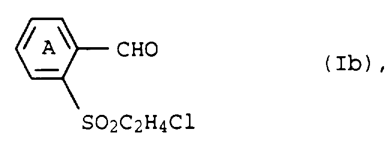 in der der Ring A jeweils die obengenannte Bedeutung besitzt.

Bevorzugt sind weiterhin Chlorethylsulfonylbenzaldehyde der Formel I, in der der Ring A unsubstituiert oder einfach durch Halogen, Nitro oder Hydroxysulfonyl substituiert ist.

Die neuen Chlorethylsulfonylbenzaldehyde der Formel I können beispielsweise in vorteilhafter Weise erhalten werden, indem man Hydroxyethylmercaptobenzaldehyde der Formel II 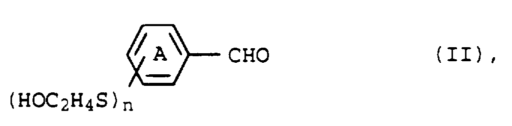 in der n und der Ring A jeweils die obengenannte Bedeutung besitzen, mit elementarem Chlor in salzsaurer Lösung bei einer Temperatur von 10 bis 80°C, vorzugsweise 30 bis 50°C, oxidiert.

Bevorzugt ist eine Verfahrensweise, bei der man die Oxidation in 0,3 bis 36 gew.-%iger, vorzugsweise 18 bis 36 gew.-%iger, Salzsäure durchführt.

Je Gewichtsteil Hydroxyethylmercaptobenzaldehyd II verwendet man in der Regel 0,5 bis 5 Gewichtsteile, vorzugweise 1 bis 3 Gewichtsteile, Salzsaure.

Je mol Hydroxyethylmercaptobenzaldehyd II verwendet man üblicherweise 2 bis 6 mol, vorzugsweise 2,5 bis 4 mol, elementares Chlor.

Das neue Verfahren wird zweckmäßig so durchgeführt, daß man eine Lösung von Hydroxyethylmercaptobenzaldehyd II in Salzsäure herstellt und darin bei der obengenannten Temperatur gasförmiges Chlor einleitet.

Nach Beendigung der Chlorzufuhr, was im allgemeinen 2 bis 6 Stunden in Anspruch nimmt, erfolgt eine Nachrührphase von ca. 3 bis 4 Stunden. Danach wird der entstandene Chlorethylsulfonylbenzaldehyd abgesaugt, mit Wasser gewaschen und getrocknet.

Die als Ausgangsprodukt dienenden Hydroxyethylmercaptobenzaldehyde sind an sich bekannt und wie vorne bereits ausgeführt, z.B. in der US-A-4 922 014 beschrieben.

Mittels des neuen Verfahrens können die neuen Chlorethylsulfonylbenzaldehyde in hoher Ausbeute und hoher Reinheit erhalten werden.

Die neuen Chlorethylsulfonylbenzaldehyde der Formel I sind wertvolle Zwischenprodukte zur Herstellung von Reaktivfarbstoffen.

Die folgenden Beispiele sollen die Erfindung näher erläutern.

### Beispiel 1

91,0 g 4-(2-Hydroxyethyl)mercaptobenzaldehyd wurden in 160 g konz. Salzsäure gelöst. In die 35 bis 40°C warme Lösung wurde bis zur Sättigung Chlor eingeleitet (ca. 4 Stunden), dann wurde 4 Stunden bei 35 bis 40°C nachgerührt bis eine dünnschichtchromatographische Analyse kein Ausgangsprodukt mehr anzeigte. Danach kühlte man unter Rühren auf Raumtemperatur ab, saugte den suspendierten 4-(2-Chlorethylsulfonyl)benzaldehyd ab, wusch mit Wasser neutral und trocknete unter vermindertem Druck bei 40°C.

Man erhielt 110 g 4-(2-Chlorethylsulfonyl)benzaldehyd vom Schmelzpunkt 70 bis 72°C (Reingehalt gemäß HPLC: 98 %).

| | |
|---|---|
| Cl_{ber.}: 15,27 % | Cl_{gef.}: 15,67 % |
| ¹H-NMR (DMSO) : δ = | 3,8 (2H), 4,0 (2H), 8,1 (4H), 10,1 (1H) ppm. |

### Beispiel 2

Man verfuhr analog Beispiel 1, führte die Oxidation jedoch in 160 g 0,4 gew.-%iger Salzsäure durch. Man erhielt 105 g 4-(2-Chlorethylsulfonyl)benzaldehyd in vergleichbarer Reinheit.

### Beispiel 3

Man verfuhr analog Beispiel 1, verwendete jedoch eine entsprechende Menge an 2-Chlor-4-(2-hydroxyethylmercapto)-benzaldehyd als Ausgangsprodukt. Man erhielt 2-Chlor-4-(2-chlorethylsulfonyl)benzaldehyd in vergleichbarer Ausbeute und Reinheit.

| | |
|---|---|
| Cl_{ber.}: 26,6 % | Cl_{gef.}: 25,73 % |
| Schmp.: 117-119°C | |
| ¹H-NMR (DMSO): δ = | 3,9 (2H), 4,1 (2H), 8,1 (2H), 8,2 (2H), 10,4 (1H) ppm. |

In analoger Weise werden die in der folgenden Tabelle aufgeführten Benzaldehyde der Formel 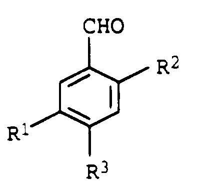 erhalten.

**Tabelle**

| Beispiel Nr. | R¹ | R | R³ |
|---|---|---|---|
| 4 | H | SO₂CH₂CH₂Cl | H |
| 5 | H | SO₂CH₂CH₂Cl | SO₂CH₂CH₂Cl |
| 6 | Cl | H | SO₂CH₂CH₂Cl |
| 7 | Cl | SO₂CH₂CH₂Cl | SO₂CH₂CH₂Cl |
| 8 | Br | H | SO₂CH₂CH₂Cl |
| 9 | NO₂ | H | SO₂CH₂CH₂Cl |
| 10 | SO₃H | H | SO₂CH₂CH₂Cl |
| 11 | NO₂ | SO₂CH₂CH₂Cl | H |
| 12 | SO₃H | SO₂CH₂CH₂Cl | H |
| 13 | H | OCH₃ | SO₂CH₂CH₂Cl |

## Patentansprüche

1. Chlorethylsulfonylbenzaldehyde der Formel I 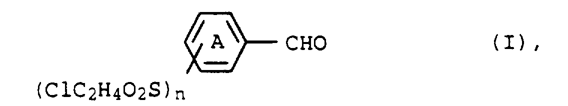 in der
n 1 oder 2 bedeutet und der Ring A substituiert sein kann.

2. Chlorethylsulfonylbenzaldehyde nach Anspruch 1, die der Formel Ia oder Ib 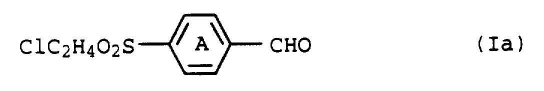 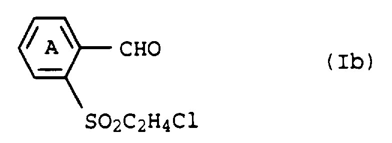 entsprechen, in der der Ring A jeweils die in Anspruch 1 genannte Bedeutung besitzt.

3. Verfahren zur Herstellung von Chlorethylsulfonylbenzaldehyden der Formel I gemäß Anspruch 1, dadurch gekennzeichnet, daß man Hydroxyethylmercaptobenzaldehyde der Formel II 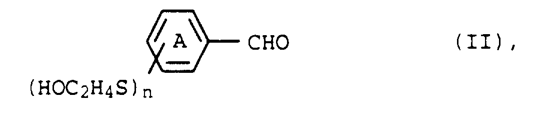 in der n und der Ring A jeweils die in Anspruch 1 genannte Bedeutung besitzen, mit elementarem Chlor in salzsaurer Lösung bei einer Temperatur von 10 bis 80°C oxidiert.

## Claims

1. Chloroethylsulfonylbenzaldehydes of the formula I 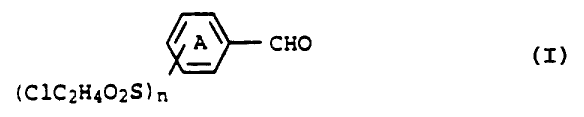 where
n is 1 or 2 and the ring A may be substituted.

2. Chloroethylsulfonylbenzaldehydes as claimed in claim 1 conforming to the formula Ia or Ib 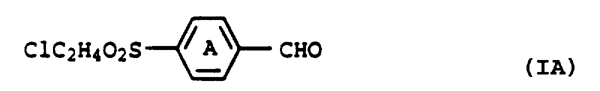 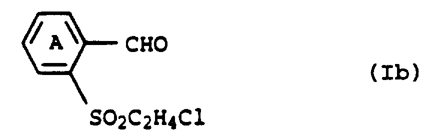 where the ring A is in either case as defined in claim 1.

3. A process for preparing chloroethylsulfonylbenzaldehydes of the formula I as claimed in claim 1, which comprises oxidizing hydroxyethylmercapto-benzaldehydes of the formula II 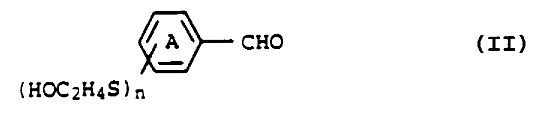 where n and the ring A are each as defined in claim 1, with elemental chlorine in hydrochloric acid solution at from 10 to 80°C.

## Revendications

1. Chloroéthylsulfonylbenzaldéhydes de formule I 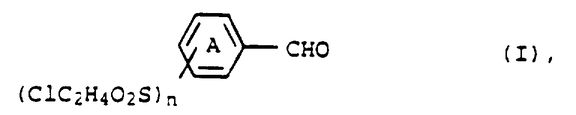 dans laquelle
n est mis pour 1 ou 2 et le noyau A peut être substitué.

2. Chloroéthylsulfonylbenzaldéhydes selon la revendication 1, qui répondent à la formule Ia ou Ib 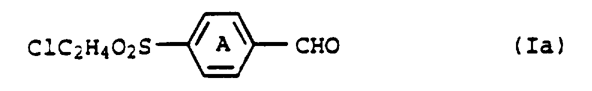 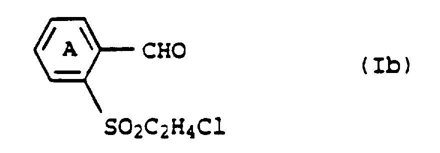 dans chacune desquelles le noyau A a la signification donnée dans la revendication 1.

3. Procédé de préparation de chloroéthylsulfonylbenzaldéhydes de formule I selon la revendication 1, caractérisé en ce que l'on oxyde des hydraxyéthylmercaptobenzaldéhydes de formule II 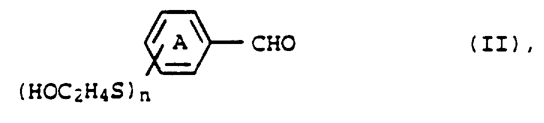 dans laquelle n et le noyau A ont chacun la signification donnée dans la revendication 1, avec du chlore élémentaire en solution dans de l'acide chlorhydrique, à une température de 10 à 80°C.
